(19) Europäisches Patentamt
European Patent Office
Office européen des brevets

(11) EP 3 216 815 A1

(12) **EUROPEAN PATENT APPLICATION**

(43) Date of publication:
**13.09.2017 Bulletin 2017/37**

(51) Int Cl.:
***C08G 63/00*** *(2006.01)* ***A61L 31/10*** *(2006.01)*

(21) Application number: **17169121.5**

(22) Date of filing: **01.03.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.03.2012 US 201213417810**

(62) Document number(s) of the earlier application(s) in accordance with Art. 76 EPC:
**13716872.0 / 2 825 581**

(71) Applicant: **Ethicon. Inc.**
**Somerville, NJ 08876 (US)**

(72) Inventors:
- **ANDJELIC, Sasa**
  **Nanuet, New York 10954 (US)**
- **JAMIOLKOWSKI, Dennis D.**
  **Long Valley, New Jersey 07853 (US)**

(74) Representative: **Carpmaels & Ransford LLP**
**One Southampton Row**
**London WC1B 5HA (GB)**

Remarks:
This application was filed on 02-05-2017 as a divisional application to the application mentioned under INID code 62.

(54) **SEGMENTED, SEMICRYSTALLINE POLY(LACTIDE-CO-EPSILON-CAPROLACTONE) ABSORBABLE COPOLYMERS**

(57) Novel semi-crystalline, segmented copolymers of lactide and *epsilon*-caprolactone exhibiting long term absorption characteristics are disclosed. The novel polymer compositions are useful for long term absorbable meshes, surgical sutures, especially monofilament sutures, and other medical devices.

EP 3 216 815 A1

## Description

### FIELD OF THE INVENTION

This invention relates to novel semi-crystalline, block copolymers of lactide and *epsilon*-caprolactone for long term absorbable medical applications, in particular, surgical sutures and hernia meshes. One use of the copolymers of the present invention is the fabrication of monofilament surgical sutures.

### BACKGROUND OF THE INVENTION

Synthetic absorbable polyesters are well known in the art. The open and patent literature particularly describe polymers and copolymers made from glycolide, L(-)-lactide, D(+)-lactide, *meso*-lactide, *epsilon*-caprolactone, p-dioxanone, and trimethylene carbonate.

A very important aspect of any bioabsorbable medical device is the length of time that its mechanical properties are retained *in vivo.* For example, in some surgical applications it is important to retain strength for a considerable length of time to allow the body the time necessary to heal while performing its desired function. Such slow healing situations include, for example, diabetic patients or bodily areas having poor or diminished blood supply. Absorbable long term sutures are known and have been made from conventional polymers, primarily from lactide. Examples include a braided suture made from a high-lactide, lactide/glycolide copolymer. Those skilled in the art will appreciate that monofilament and multifilament bioabsorbable sutures exist and that short term and long term bioabsorbable sutures exist. Long term functioning may be described as retaining a certain amount of mechanical integrity *in vivo* beyond 10 to 12 weeks post implantation.

What does not presently exist is a bioabsorbable polymer that can be made into a suture having a monofilament construction that is soft enough to provide superior handling characteristics to the surgeon, yet maintain its properties post-implantation to function long term. There then remains the problem of providing such a polymer. There is also a need for an absorbable surgical suture made from such a polymer. Absorbable sutures generally come in two basic forms, multifilament braids and monofilament fibers. For a polymer to function as a monofilament, it must generally possess a glass transition temperature, Tg, below room temperature. A low $T_g$ helps to insure a low Young's modulus which in turn leads to filaments that are soft and pliable. A high $T_g$ material would likely result in a wire-like fiber that would lead to relatively difficult handling monofilament sutures; in this art, such sutures would be referred to or described as having a poor "hand". If a polymer possesses a high $T_g$, and it is to be made into a suture, it invariably must be a construction based on multifilament yarns; a good example of this is a braid construction. It is known that monofilament sutures may have advantages over multifilament sutures. Advantages of monofilament structures include a lower surface area, with less tissue drag during insertion into the tissue, with possibly less tissue reaction. Other advantages include no wicking into interstices between filaments in which bacteria can move and locate, and potentially form biofilms. There is some thought that infectious fluids might more easily move along the length of a multifilament construction through the interstices by a wicking action; this of course cannot happen in monofilaments. In addition, monofilament fiber is generally easier to manufacture as there are no braiding steps usually associated with multifilament yarns.

It is to be understood that these polymers would also be useful in the construction of fabrics such as surgical meshes. Besides opportunities in long term sutures and meshes, there exist opportunities for such polymers in devices that must be made from a deformable resin, ideally fabricated by known and conventional methods, including for example injection molding.

Crystalline block copolymers of *epsilon*-caprolactone andp-dioxanone are disclosed in US 5,047,048. The copolymers range from about 5 to about 40 weight percent *epsilon*-caprolactone and the absorption profile is similar to poly(*p*-dioxanone). Absorbable surgical filaments made from these copolymers have a tensile strength similar to poly(*p*-dioxanone) with better pliability than poly(*p*-dioxanone) and a lower Young's modulus of elasticity. The described copolymers are random copolymers.

It is expected that fibers made from the *epsilon*-caprolactone/*p*-dioxanone copolymers, rich in *p*-dioxanone, using the teachings of '048 will retain their mechanical properties post-implantation similar to *p*-dioxanone homopolymer. Surgical sutures made from these copolymers are expected to have virtually no mechanical strength remaining after about 8 to 10 weeks *in vivo.* There then remains a need for a material that could retain mechanical properties significantly longer than that exhibited by the copolymers of '048 and that would possess Young's moduli low enough to allow fabrication into soft monofilament fibers useful as suture or mesh components.

US 5,314,989, entitled "Absorbable Composition", describes a block copolymer for use in the fabrication of bioabsorbable articles such as monofilament surgical sutures. The copolymer is prepared by copolymerizing one or more hard phase forming monomers and 1,4-dioxan-2-one, and then polymerizing one or more hard phase forming monomers with the dioxanone-containing copolymer. The (co)polymer of '989 will not result in monofilament fibers possessing long term strength; i.e., strength beyond 8 to 10 weeks post-implantation.

Similarly, US 5,522,841, entitled "Absorbable Block Copolymers and Surgical Articles Fabricated Therefrom", describes absorbable surgical articles formed from a block copolymer having one of the blocks made from hard phase forming monomers and another of the blocks made from random copolymers of soft phase forming monomers. The soft phase of the claimed copolymers of ’841 requires the inclusion of polyalkylene oxide segments.

US 5,705 181, entitled "Method of Making Absorbable Polymer Blends of Polylactides, Polycaprolactone and Polydioxanone", describes absorbable binary and tertiary blends of homopolymers and copolymers of poly(lactide), poly(glycolide), poly(*epsilon*-caprolactone), and poly(p-dioxanone). These materials are blends and not copolymers.

US 5,133,739 describes block copolymers prepared from and glycolide having a hard phase. US 2009/0264040A1 describes melt blown nonwoven materials prepared from caprolactone/glycolide copolymers. Although both of these disclosures are directed towards absorbable materials containing polymerized caprolactone, the materials absorb rather quickly and thus are not useful for long term implants.

US 5,797,962 discloses copolymers of lactide and *epsilon*-caprolactone, but these are random in nature. For a given (polymerized) monomer composition, random copolymers in this class of materials (polyesters based on ring-opened lactones) exhibit reduced crystallinity levels, limiting their ability to remain dimensionally stable when exposed to temperatures above their glass transition temperatures, when compared to corresponding block copolymers.

US 6,342,065 discloses copolymers of lactide and *epsilon*-caprolactone with the lactide component of 86% or higher. One major disadvantages of such high lactide content is very high Young’s modulus (due to high Tg value), which is often unsuitable for certain medical uses; these include monofilament applications.

The paper entitled, "Synthesis, characterization and melt spinning of a block copolymer of L-lactide and *epsilon*-caprolactone for potential use as an absorbable monofilament surgical suture", authored by Y. Baimark, R. Molloy, N. Molloy, J. Siripitayananon, W. Punyodom, and M. Sriyai, [reference: Journal of Materials Science: Materials In Medicine 16 (2005) 699-707] describes a copolymer with an overall final composition of 79/21 lactide and *epsilon*-caprolactone with a pre-polymer composition of 52/48 lactide and *epsilon*-caprolactone (mole basis, in each case). Additionally, the prepolymer composition is semi-crystalline which adversely affects the fiber handling. Because of these characteristics, this material is expected to be quite stiff and thus unsuitable for use as a monofilament surgical suture.

There is a need in this art for novel, long term bioabsorbable sutures that have good handling characteristics and strength retention *in vivo.* There is a further need in this art for novel bioabsorbable polymer compositions for manufacturing such sutures and other bioabsorbable medical devices.

## SUMMARY OF THE INVENTION

Novel semi-crystalline, block copolymers of lactide and *epsilon*-caprolactone for long term absorbable medical applications are disclosed. The semicrystalline absorbable segmented copolymers, have repeating units of polymerized lactide and polymerized *epsilon*-caprolactone. The mole ratio of polymerized lactide to polymerized *epsilon*-caprolactone is between about 60:40 to about 75:25, and the copolymers possess a first heat $T_g$ as determined by differential scanning calorimetry at a scan rate of 10°C per minute, equal to or less than 0°C, and a crystallinity level of about 25 percent to about 50 percent, as measured by wide angle X-ray diffraction. The copolymers also have an inherent viscosity at least about 0.5 dL/g, as measured in a 0.1 g/dl solution of HFIP at 25°C.

Another aspect of the present invention is a bioresorbable copolymer of the structure A-B-A. The end-segments A of this copolymer consist of polymerized lactide blocks and the middle segment B consists of a polymerized lactide-co-*epsilon*-caprolactone block. The middle segment B represents about 25 weight percent to about 60 weight percent of the copolymer.

A further aspect of the present invention is a bioabsorbable, semi-crystalline segmented copolymer comprising the reaction product of (a) a pre-polymer formed from polymerizing lactide monomer, and *epsilon*-caprolactone monomer in the presence of an initiator and preferably a suitable amount of catalyst, wherein the mole ratio of lactide to *epsilon*-caprolactone in the prepolymer is between about 45:55 to about 30:70; and, (b) lactide monomer.

Yet another aspect of the present invention is a long term bioabsorbable suture made from one of the above-described copolymers.

Another aspect of the present invention is a bioabsorbable medical device made from one of the above described copolymers.

Still yet another aspect of the present invention is a method of manufacturing a medical device from said novel copolymers.

An additional aspect of the present invention is a medical device injection molded from one of the above-described copolymers, wherein the device is deformable.

A further aspect of the present invention is a method of performing a surgical procedure wherein a medical device made from the novel copolymers of the present invention is implanted in tissue in a patient.

These and other aspects and advantages of the present invention will become more apparent from the following description and accompanying drawings.

## BRIEF DESCRIPTION OF THE DRAWINGS

FIG. 1 is a plot of isothermal crystallization kinetics, as measured by Differential Scanning Calorimetry, of the final inventive copolymers of Examples 1 and 3A.

FIG. 2 is histogram of sequence distribution results for the final inventive copolymers of Examples 1, 2A, 2B, 3A and 3B as measured by $^{13}C$ NMR.

## DETAILED DESCRIPTION OF INVENTION

For clarity purposes, we will define a number of terms. We will define a random (copolyester) copolymer as a copolyester having a sequence distribution of the monomer moieties along the chain that is at least as random as a copolymer of that overall composition made from lactone monomers or hydroxy acids in which all the monomers are added in a single step to the polymerization reactor, and as governed by reactivity ratio considerations at the time of the polymerization.

Statistical copolymers are copolymers in which the sequence of monomer residues follows a statistical rule. If the probability of finding a given type monomer residue at a particular point in the chain is equal to the mole fraction of that monomer residue in the chain, then the polymer may be referred to as a "truly random copolymer". In a random copolymer, the sequence distribution of monomeric units follows Bernoullian statistics.

Truly random copolymers are difficult to find due to the complications of the phenomena of monomer reactivity ratios. Although the monomers may be added to a batch reactor in a single step, there may be a slight propensity of one monomer adding to the growing chain over another monomer. This will be discussed further in this specification.

To form a random copolymer, in a batch polymerization process, the monomers are generally added to the batch reactor in a single step. In a continuous polymerization process, the monomers are added to the continuous reactor in a substantially constant composition.

A segmented (copolyester) copolymer on the other hand possesses a non-random sequence distribution beyond what would be expected based on reactivity ratio considerations that is less random than a random copolymer.

When the sequence length of a given monomer starts to get large, one begins to approach a blocky structure. A "block copolymer" can be multi-block in nature, tetrablock, triblock or diblock, depending on the number of different chemical blocks.

A block copolymer that is a "diblock copolymer" might have a structure containing two different chemical blocks and is then referred to as an A-B block copolymer. If a triblock copolymers has one monomer sequence, A, at its ends and a second, B, in its interior, it might be referred to an A-B-A block copolymer.

A technique to produce a non-random sequence distribution in ring-opening polymerizations is the method of adding different monomer feeds to the reactor in stages. One might add an amount of monomer B to the reactor with a monofunctional initiator. A polymer is formed made of only B sequences. A second monomer, A, is then added to the reactor; the copolymer thus formed might then be an A-B block copolymer. Alternately, if one used a difunctional initiator at the start of the polymerization, the copolymer thus formed might then be an A-B-A block copolymer.

To help in characterizing the "blockiness" of the sequence distribution of a copolymer, Harwood (reference: Harwood, H. J.; Ritchey, W. M. Polymer Lett. 1964, 2, 601) introduced a "run number" concept. For a copolymer made up of polymerized "A" repeat units and polymerized "B" repeat units, the corresponding run numbers reflect the average chain sequence length for the individual "monomers". In looking down the chain, every time one encountered an A unit, a counter was activated. Every time one came across another A unit, the counter was increased by one; the counter was stopped as soon as a B unit was reached. When the entire chain is sampled and the work completed on the rest of resin, one can establish an average value for the Harwood run number for the "A" unit. The same can be done for "B". Statistical treatments have shown that for a theoretically random copolymer of A/B molar composition, the Harwood run number for each of the components can be calculated based on the following equations:

$$HRN_A = 1 + ([A]/[B]) \text{ and } HRN_B = 1 + ([B]/[A]) \qquad (1)$$

where $HRN_A$ and $HRN_B$ are the Harwood Run Numbers for repeat units A and B, respectively, [A] and [B] are the molar fractions of repeat units A and B, respectively.

Thus a 20/80 A/B random copolymer made up of A and B units is expected to have Harwood run numbers of 1.25 and 5.0 for A and B, respectively. Let us now address non-random copolymers. One might have a copolymer of the same 20/80 composition with a Harwood run number for the A component much higher than the 1.25 value displayed in the random copolymer, for instance 1.5 or 3. This is clearly indicative of a propensity of "A" units to be together - a blocky sequence distribution.

In a copolymerization, the monomers may not be sequenced exactly randomly due to a phenomenon in which there is a great propensity of monomer 1 to add to a growing chain terminated in a "monomer 1 repeat unit" or a great propensity of monomer 1 to add to a growing chain terminated in a "monomer 2 repeat unit". The concept of reactivity ratios, $r_1$ and $r_2$, has been developed to describe the phenomena. Specifically, the Mayo-Lewis equation, also called the copolymerization equation in polymer chemistry describes the distribution of monomers in a copolymer. Taking into consideration a monomer mix of two components $M_1$ and $M_2$ and the four different reactions that can take place at the reactive chain end terminating in either monomer ($M^*$) with their reaction rate constants *k*:

$$M^*_1 + M_1 \xrightarrow{k_{11}} M_1M^*_1 \quad (2)$$

$$M^*_1 + M_2 \xrightarrow{k_{12}} M_1M^*_2 \quad (3)$$

$$M^*_2 + M_1 \xrightarrow{k_{21}} M_2M^*_1 \quad (4)$$

$$M^*_2 + M_2 \xrightarrow{k_{22}} M_2M^*_2 \quad (5)$$

Reactivity ratios are defined as:

$$r_1 = (\boldsymbol{k}_{11}/\boldsymbol{k}_{12}) \quad (6)$$

$$r_2 = (\boldsymbol{k}_{22}/\boldsymbol{k}_{21}) \quad (7)$$

where $k_{11}$, $k_{12}$, $k_{21}$, and $k_{22}$ are the rate constants of the reactions shown in equations 2 through 5, respectively.

A statistical random copolymer is generally formed when the values of $r_1$ and $r_2$ are both one. The reactivity ratio that corresponds to *epsilon*-caprolactone monomer adding to a chain terminated in a lactidyl moiety (i.e. polymerized L(-)-lactide sequence) has been experimentally determined to be 44 while L(-)-lactide monomer adding to a chain terminated in a caproyl moiety (i.e., polymerized *epsilon*-caprolactone sequence) is 0.28. Since the two reactivity ratios are quite different, this then leads to copolymers with a slightly non-random sequence distribution, even when both monomers are added to the reactor together at the start of the polymerization.

For a given copolymer there are expected Harwood run numbers associated with each of the polymerized monomers, assuming the sequence is truly random in nature. There is also an experimentally determined average chain sequence length value for each of the components. We define herein a "Randomness Factor" for each of the polymerized monomers; we abbreviate it as $RF_x$, where x denotes the particular monomer under consideration. The $RF_x$ for monomer x is the ratio of the experimentally determined average sequence length and the corresponding Harwood Run Number.

For instance, in the 20/80 A/B random copolymer made up of A and B units described earlier, if it was indeed statically random, the expected Harwood run numbers should be 1.25 and 5.0 for A and B, respectively. If experimentally it was found that the average chain sequence length values for components A and B were 1.88 and 8.50 respectively, then one could calculate an $RF_A$ Value of 1.5 (= 1.88/1.25) and an $RF_B$ value of 1.7 (= 8.5/5.0). Again the "randomness factor" is calculated from the ratio of the experimentally determined average chain sequence length and the corresponding theoretical Harwood Run Number assuming a statistically random sequence distribution.

An example of a random (copolyester) copolymer made from lactone monomers is the combining of 70 moles of lactide and 30 moles of epsilon-caprolactone into a reactor and polymerizing the combination without introducing any additional monomer in a subsequent step. It should be noted that a random (copolyester) copolymer made from lactide and *epsilon*-caprolactone in the compositional range of 60/40 to 75/25 will possess only very low levels of crystallinity, i.e., be nearly amorphous. Such lactide / *epsilon*-caprolactone copolymers possessing low levels of crystallinity will be unsuitable for use as strong fibers due to a lack of dimensional stability in view of the high orientation needed to achieve high strength. It should also be noted that random (copolyester) copolymers of even moderate molecular weight, made from lactide and *epsilon*-caprolactone in the compositional range of 60/40 to 75/25, will possess glass transition temperatures greater than room temperature, leading to stiff articles.

An example of a non-random (copolyester) copolymer made from lactone monomers or hydroxy acids is one in which the monomers are added to the reactor sequentially. One might add, in a first stage of the polymerization, 70 moles of lactide and 30 moles of epsilon-caprolactone to the reactor and polymerize this mixture; after the subsequent formation of the "prepolymer", an additional portion of one of the monomers, or a third monomer, is added. The sequence distribution of monomers along the various chains is then purposefully controlled.

The terms absorbable, bioabsorbable, bioresorbable, resorbable, biodegradable are used herein interchangeably.

The final copolymer of the present invention is semi-crystalline, while the prepolymer is amorphous. With the prepolymer compositions being in the range of 45/55 to 30/70 and the final compositions 60/40 to 75/25, mole basis, L(-)-lactide /*epsilon*-caprolactone, we have unexpectedly discovered that the copolymers of the present invention are semi-crystalline in nature with glass transition temperatures well below room temperature. One possible application for such polymers is in the production of novel, strong soft fibers.

Poly(lactide) is a high glass transition ($T_g$ of 65°C), semi-crystalline polyester. This material has a high elastic modulus and is thus quite stiff making it generally unsuitable for monofilament surgical sutures. In addition, it does not absorb quickly enough for many key surgical applications, i.e., it lasts too long *in vivo.* We have found, however, that certain lactide and *epsilon*-caprolactone copolymers are, surprisingly and unexpectedly, particularly useful for the applications requiring both softness and a longer term mechanical property loss profile.

For instance, a 72/28 mol/mol poly(lactide-co-*epsilon*-caprolactone) copolymer [72/28 Lac/Cap] was prepared in a sequential addition type of polymerization starting with a first stage charge of lactide and *epsilon*-caprolactone charge (45/55 Lac/Cap mole percent) followed by a subsequent second stage of lactide addition only. The total initial charge was 75/25 mol/mol lactide/*epsilon*-caprolactone. Due to incomplete conversion of monomer-to-polymer and difference in reactivity, it is not uncommon to have the final (co)polymer composition differ from the feed composition. The final composition of the copolymer was found to be 72/28 mol/mol lactide/*epsilon*-caprolactone. See Example 2A for the details of this copolymerization.

The present invention is directed toward copolymers of lactide and *epsilon*-caprolactone. More specifically, this class of copolymers rich in lactide and made to have a blocky sequence distribution, that is non-random. In such lactide/*epsilon*-caprolactone copolymers in which the majority of the material is based on lactide, the morphology of the resin needs to be optimized to be useful in long term applications. We have discovered that the compositions must be rich in lactide, e.g., having a polymerized lactide content of 50 percent or greater.

We have unexpectedly discovered novel bioabsorbable polymers having a relatively narrow composition range and a non-random sequence distribution, which when made into a monofilament fiber will yield a suture that is soft enough to have good handling characteristics, yet possesses sufficiently effective mechanical integrity *in vivo* beyond 10 to 12 weeks post implantation. Segmented, that is possessing a non-random sequence distribution beyond what would be expected based on reactivity ratio considerations, poly(lactide-co-*epsilon*-caprolactone) copolymers comprising a polymerized lactide having a molar level between 60 to 75 percent and a polymerized *epsilon*-caprolactone molar level between 25 to 40 percent are useful in the practice of the present invention. This class of copolymers, the poly(lactide-co-*epsilon*-caprolactone) family rich in lactide, ideally contains about 25 to about 35 mole percent of polymerized *epsilon*-caprolactone.

Specifically, poly(lactide-co-*epsilon*-caprolactone) copolymers rich in polymerized lactide having levels of incorporated lactide lower than about 60 mole percent are unsuitable for copolymers of the present invention because of crystallization difficulties. On the other hand, we have found that poly(lactide-co-*epsilon*-caprolactone) copolymers rich in polymerized lactide having levels of incorporated lactide greater than about 75 mole percent are unsuitable due to high modulus and too long absorption times.

Dimensional stability in a fiber used to manufacture a surgical suture is very important to prevent shrinkage, both in the sterile package before use, as well as in the patient after surgical implantation. Dimensional stability in a low $T_g$ material can be achieved by crystallization of the formed article. Regarding the phenomena of crystallization of copolymers, a number of factors play important roles. These factors include overall chemical composition and sequence distribution.

Although the overall level of crystallinity (and the $T_g$ of the material) plays a role in dimensional stability, it is important to realize that the rate at which the crystallinity is achieved is critical to processing. If a lower Tg material is processed and its rate of crystallization is very slow, it is very difficult to maintain dimensional tolerances since shrinkage and warpage easily occur. Fast crystallization is thus an advantage. We have discovered that for the systems at hand, to increase the rate of crystallization of a copolymer of given overall chemical composition, a block structure would be preferable over a random sequence distribution. However, surprisingly and unexpectedly, the inventors were able to achieve this with the two lactone monomers, for instance lactide and *epsilon*-caprolactone, notwithstanding experimental difficulties and challenges due to transesterification and other factors.

According to the present invention, the compositional sequence of the inventive semi-crystalline copolymer is schematically illustrated as follows:

LLLLLLLLLLLLLL-CLCLCCLCLCLCCCLCLCCLC-LLLLLLLLLLLLLL Polymerized Lactide Block-Polymerized (Lactide-co-*epsilon*-Caprolactone)-Polymerized Lactide Block

with the semi-crystalline polylactide blocks representing approximately 45 to 70 weight percent of the copolymer and with the middle block formed from a nearly amorphous random prepolymer based on polymerized lactide and *epsilon*-caprolactone. In the above formula, L represents lactide, and C represents *epsilon*-caprolactone.

The novel copolymers of the present invention are prepared by first polymerizing the lactide and *epsilon*-caprolactone monomers at temperatures between about 170°C and about 240°C. Temperatures between about 185°C and about 195°C are particularly advantageous. Although a monofunctional alcohol such as dodecanol might be used for initiation, a diol such as diethylene glycol has been found to work well. Combinations of mono-functional and di-functional, or multifunctional conventional initiators may also be used as a means of further influencing some important characteristics such as morphological development including crystallization rates and ultimate crystallinity levels. Reaction times can vary with catalyst level. Suitable catalysts include conventional catalysts such as stannous octoate. Sufficiently effective amounts of catalyst are utilized. The catalyst may be used at an overall monomer / catalyst level ranging from about 10,000/1 to about 300,000/1, with a preferred level of about 25,000/1 to about 100,000/1. After the completion of this first stage of the polymerization (e.g., 4 to 6 hours), the temperature is raised to above 200°C (typically 205 to 210°C). Once the temperature is increased, for example to 205°C, the balance of lactide monomer can be added to the reactor; this can be conveniently done by pre-melting the monomer and adding it in a molten form. Once the second portion of lactide monomer is added, the temperature is brought to about 190°C to about 200°C to complete the co-polymerization (e.g., 1 to 2 hours).

It will be clear to one skilled in the art that various alternate polymerization approaches and parameters are possible to produce the copolymers of the present invention. For example, although not preferred, it may be possible to conduct all or part of the polymerizations without a catalyst present.

It is to be understood that the monomer feed added to the prepolymer may not necessarily need to be pure lactide. Instead of adding pure lactide monomer to the prepolymer, up to about ten mole percent of another monomer may be used to adjust the monomer feed added to the prepolymer. For instance, the monomer feed added to the prepolymer may contain minor amounts of *epsilon*-caprolactone; the monomer feed might be for instance 90/10 lactide/*epsilon*-caprolactone. Adding *epsilon*-caprolactone to the "end blocks" will lower the melting point, crystallization rate and overall crystallinity of the final copolymer. Adding more than about ten mole percent reduces properties too much to be useful for most applications. The compositional sequence of this variant of the inventive semi-crystalline copolymer is schematically illustrated as follows:

LLCLLLLLLLLCLL-CLCLCCLCLCLCCCLCLCCLC-LLLLLLLCLLLLLL

In certain embodiments, one may wish to add minor amounts of glycolide to the monomer feed added to the prepolymer. For instance, the monomer feed added to the prepolymer may contain up to about ten mole percent glycolide; the monomer feed might be for instance 90/10 lactide/glycolide. Adding glycolide to the "end blocks" will lower the melting point, crystallization rate and overall crystallinity of the final copolymer, as well as increase the rate of absorption of the copolymer. Again adding more than about ten mole percent reduces properties too much to be useful for most applications. The compositional sequence of this variant of the inventive semi-crystalline copolymer is schematically illustrated as follows:

LLLLLGLLLLLLLL-CLCLCCLCLCLCCCLCLCCLC-LLLLGLLLLLGLLL

In the above formula, L represents lactide, and C represents *epsilon*-caprolactone, and G represents glycolide.

It is also to be understood that slight modification of the first stage prepolymer monomer feed composition can be adjusted to provide certain desired characteristics, all within the scope of the present invention. Thus other lactones such as p-dioxanone, trimethylene carbonate, or glycolide might be added to the lactide and *epsilon*-caprolactone mixture of the first stage. The amount of another monomer that is added in this first stage might be up to approximately or about 20 mole percent to adjust properties. For instance adding small amounts of glycolide to the lactide and *epsilon*-caprolactone in the first stage prepolymer monomer feed will decrease the breaking strength retention profile of a suture; this may occur without affecting the crystallization rate or overall crystallinity of the final copolymer. The compositional sequence of this variant of the inventive semi-crystalline copolymer is illustrated as follows:

LLLLLLLLLLLLL-CLGLCCLCLCLCGCLCLCCGC-LLLLLLLLLLLL

Polymerization variations include the possibility of adding the "second stage" monomer to the prepolymer in multiple steps. Alternately, one might then add additional monomer to the formed prepolymer in a continuous fashion over a short period of time, for instance 10 minutes or over a relatively longer period of time, for instance 2 hours.

Although we describe adding all of the catalyst at the start of the polymerization, that is, at the start of the formation of the prepolymer, one may only add a portion of the catalyst in this stage of the polymerization, adding the remainder later, during the introduction of the monomer to the now formed prepolymer.

It is to be understood that that sufficiently effective amounts of acceptable coloring agents such as dyes and pigments might be added at any stage of the polymerization. Such colorants include D&C Violet № 2 or D&C Green № 6.

The present invention can be practiced using the L(-) isomer of lactide monomer, L(-)-lactide, or the D(+)isomer, D(+)-lactide. A mixture of the two monomers may be used, provided the resulting final copolymer crystallizes sufficiently to the extent needed to effectively provide dimensional stability. One may then use a isomer lactide monomer blend corresponding to 95 percent L(-)-lactide and 5 percent D(+)-lactide. Alternately, one may use a 50/50 mixture of the L and D isomers [a racemic mixture], in combination with an appropriate level of *epsilon*-caprolactone to form the prepolymer, but use only L(-)-lactide [or D(+)-lactide] in the monomer feed to be introduced into the formed prepolymer. A copolymer so produced of the present invention will be semicrystalline in nature.

It is to be understood that low temperature polymerization techniques may also be used to make the copolymers of the present invention. As an example, the reaction is maintained at the melt reaction temperature for some period of time (e.g., 3 to 4 hours), followed by the discharge of the reaction product into suitable containers for subsequent low temperature polymerization (e.g., 120°C) for an extended period of time sufficient to effectively complete the co-polymerization. Higher monomer-to-polymer conversions may be possible utilizing this alternate low temperature finishing approach.

Again, one skilled in the art can provide a variety of alternate polymerization schemes to provide the novel copolymers of the present invention.

The novel copolymers of the present invention are semicrystalline in nature, having a crystallinity level ranging from about 25 to about 50 percent. They will have a molecular weight sufficiently high to allow the medical devices formed therefrom to effectively have the mechanical properties needed to perform their intended function. For melt blown nonwoven structures and microsphere formation, the molecular weights may be a little lower, and for conventional melt extruded fibers, they may be a little higher. Typically, for example, the molecular weight of the copolymers of the present invention will be such so as to exhibit inherent viscosities as measured in hexafluoroisopropanol (HFIP, or hexafluoro-2-propanol) at 25°C and at a concentration of 0.1 g/dL between about 0.5 to about 2.5 dL/g.

Surgical sutures made from the novel copolymers of the present invention preferably are monofilaments with a Young's modulus of less than about 300,000 psi. Although monofilament fibers are a focus of the present invention, it is to be understood that the multifilament yarns can be spun from the inventive copolymers. The resulting yarns can be used to provide surgical braided sutures as well as surgical mesh constructs and other fabric-based products. Surgical mesh products can be fabricated from the monofilament fibers made from the polymers of the present invention. When such mesh products are constructed from monofilament fibers, fiber diameters between about 1 and about 10 mils find particular utility. In one embodiment, the heat treated copolymer has a glass transition temperature on the first heat as measured by DSC (heating rate 10°C/min) below about -5°C. The novel copolymers of the present invention will preferably have an absorption time between about 12 and about 24 months *in vivo.*

In one embodiment, medical devices made of the copolymers of the present invention may contain sufficiently effective amounts of conventional active ingredients or may have coatings containing such ingredients, such as antimicrobials, antibiotics, therapeutic agents, hemostatic agents, radio-opaque materials, tissue growth factors, and combinations thereof. In one embodiment the antimicrobial is Triclosan, PHMB, silver and silver derivatives, or any other bioactive agent.

The variety of therapeutic agents that may be used is vast. In general, therapeutic agents which may be administered via these medical devices and compositions of the present invention include, without limitation, antiinfectives, such as antibiotics and antiviral agents; analgesics and analgesic combinations; anorexics; antihelmintics; antiarthritics; antiasthmatic agents; adhesion preventatives; anticonvulsants; antidepressants; antidiuretic agents; antidiarrheals; antihistamines; anti-inflammatory agents; antimigraine preparations; contraceptives; antinauseants; antineoplastics; antiparkinsonism drugs; antipruritics; antipsychotics; antipyretics, antispasmodics; anticholinergics; sympathomimetics; xanthine derivatives; cardiovascular preparations including calcium channel blockers and beta-blockers such as pindolol and antiarrhythmics; antihypertensives; diuretics; vasodilators, including general coronary, peripheral and cerebral; central nervous system stimulants; cough and cold preparations, including decongestants; hormones, such as estradiol and other steroids, including corticosteroids; hypnotics; immunosuppressives; muscle relaxants; parasympatholytics; psychostimulants; sedatives; tranquilizers; naturally derived or genetically engineered proteins, polysaccharides, glycoproteins, or lipoproteins; oligonucleotides, antibodies, antigens, cholinergics, chemotherapeutics, hemostatics, clot dissolving agents, radioactive agents and cystostatics. Therapeutically effective dosages may be determined by *in vitro* or *in vivo* methods. For each particular additive or active ingredient, individual determinations may be made to determine the optimal dosage required. The determination of effective dosage levels to achieve the desired result will be within the realm of one skilled in the art. The release rate of the additives or active ingredients may also be varied within the realm of one skilled in the art to determine an advantageous profile, depending on the therapeutic conditions to be treated.

The copolymers of the subject invention can be melt extruded by a variety of conventional means. Monofilament fiber formation can be accomplished by melt extrusion followed by extrudate drawing with or without annealing. Multifilament fiber formation is possible by conventional means. Methods of manufacturing monofilament and multifilament braided sutures are disclosed in U.S. Patent No. 5,133,739, entitled "Segmented Copolymers of *epsilon*-Caprolactone and Glycolide" and U.S. Patent No. 6,712,838 entitled "Braided Suture with Improved Knot Strength and Process to Produce Same", which are incorporated by reference herein in their entirety.

The copolymers of the present invention may be used to manufacture conventional medical devices in addition to sutures using conventional processes. For example, injection molding may be accomplished after allowing the copolymer to crystallize in the mold; alternately, biocompatible nucleating agents might be added to the copolymer to reduce cycle time. The copolymers of the present invention may be used to manufacture medical devices that function in part by being deformable without undergoing significant fracturing, cracking, splintering or other forms of breakage. Medical devices that function in part by being deformable include those that have hinges or are required to bend substantially. The medical devices may include (but are not limited to), conventional medical devices, especially implantable medical devices, including staples, tacks, clips, sutures, barbed sutures, tissue fixation devices, mesh fixation devices, anastomosis devices, suture and bone anchors, tissue and bone screws, bone plates, prostheses, support structures, tissue augmentation devices, tissue ligating devices, patches, substrates, meshes, tissue engineering scaffolds, drug delivery devices, and stents, etc.

It is to be understood that the copolymers of the present invention may be used to make fabrics via conventional melt blown nonwoven techniques. In addition, due to the expected good solubility in common organic solvents of the copolymers of the present invention, useful medical devices can be made by electrostatic spinning techniques. Similarly, the copolymers of the present invention may also be used to manufacture microcapsules and microspheres; these may be made to contain therapeutic agents for delivery to the patient.

Sutures made from the copolymers of the present invention may be used in conventional surgical procedures to approximate tissue or affix tissue to medical devices. Typically, conventional surgical needles are affixed to one or both ends of the sutures. Typically, after a patient is prepared for surgery in a conventional matter, including swabbing the outer skin with antimicrobial solutions and anesthetizing the patient, the surgeon will make the required incisions, and after performing the required procedure proceed to approximate tissue using the long-term absorbable sutures of the present invention (in particular monofilament sutures) made from the novel copolymers of the present invention using conventional suturing techniques and equivalents thereof. In addition to tissue approximation, the sutures may be used to affix implanted medical devices to tissue in a conventional manner. The sutures may be utilized for other conventional procedures including vessel occlusion, vessel anastomosis, duct closure, tissue and organ support, medical device affixation, etc. After the incisions are approximated, and the procedure is completed, the patient is then moved to a recovery area. The long-term absorbable sutures of the present invention after implantation in the patient sufficiently retain their strength *in vivo* for the required time to allow effective healing and recovery.

The following examples are illustrative of the principles and practice of the present invention, although not limited thereto.

## EXAMPLE 1

### Synthesis of Segmented Block Copolymer Poly(L(-)-lactide-co-*epsilon*-caprolactone) at 64/36 by Mole [Initial Feed Charge of 70/30 Lac/Cap]

Using a conventional 2-gallon stainless steel oil-jacketed reactor equipped with agitation, 1,520 grams of *epsilon*-caprolactone and 1,571 grams of L(-)-lactide were added along with 3.37 grams of diethylene glycol and 2.34 mL of a 0.33M solution of stannous octoate in toluene. After the initial charge, a purging cycle with agitation at a rotational speed of 10 RPM in a downward direction was initiated. The reactor was evacuated to pressures less than 150 mTorr followed by the introduction of nitrogen gas. The cycle was repeated once again to ensure a dry atmosphere. At the end of the final nitrogen purge, the pressure was adjusted to be slightly above one atmosphere. The rotational speed of the agitator was reduced to 7 RPM in a downward direction. The vessel was heated by setting the oil controller at 190°C. When the batch temperature reached 110°C, rotation of the agitator was switched to an upward direction. The reaction continued for 4.5 hours from the time the oil temperature reached 190°C.

After the completion of the first stage portion of the polymerization, a very small amount of resin was discharged for analysis purposes; selected characterization was performed. The chemical composition of the prepolymer, as determined by NMR, was 45 mole percent polymerized lactide and 55 mole percent polymerized caprolactone with about 2 percent of residual unreacted monomer. The DSC data revealed that the prepolymer was fully amorphous with no crystallinity developed even after heat treatment. The glass transition temperature was determined to be -17°C (minus 17°C).

In the second stage portion of the polymerization, the heating oil controller set point was raised to 205°C, and 2,909 grams of molten L(-)-lactide monomer was added from a melt tank with the agitator speed of 12.5 RPM in a downward direction for 15 minutes. The agitator speed was then reduced to 7.5 RPM in the downward direction. The oil controller was then decreased to 200°C and the reaction proceeded an additional 2.5 hours prior to the discharge.

At the end of the final reaction period, the agitator speed was reduced to 2 RPM in the downward direction, and the polymer was discharged from the vessel into suitable containers. Upon cooling, the polymer was removed from the containers and placed into a freezer set at approximately -20°C for a minimum of 24 hours. The polymer was then removed from the freezer and placed into a Cumberland granulator fitted with a sizing screen to reduce the polymer granules to approximately 3/16 inches in size. The granules were sieved to remove any "fines" and weighed. The net weight of the ground and sieved polymer was 5.065 kg; the ground polymer was then placed into a 3 cubic foot Patterson-Kelley tumble dryer to remove any residual monomer.

The Patterson-Kelley tumble dryer was closed, and the pressure was reduced to less than 200 mTorr. Once the pressure was below 200 mTorr, the dryer rotation was activated at a rotational speed of 10 RPM with no heat for 18 hours. After the 18 hour period, the oil jacket temperature was set to 55°C with drying at this temperature for 4 hours. The oil temperature was again raised, this time to 65°C; this period lasted 2 hours. Two additional heating periods were employed: 85°C for 12 hours, and 110°C for 3 hours. At the end of the final heating period, the batch was allowed to cool for a period of 4 hours while maintaining rotation and vacuum. The polymer was discharged from the dryer by pressurizing the vessel with nitrogen, opening the discharge valve, and allowing the polymer granules to descend into waiting vessels for long term storage.

The long term storage vessels were air tight and outfitted with valves allowing for evacuation so that the resin was stored under vacuum. The dried resin exhibited an inherent viscosity of 1.27 dL/g, as measured in hexafluoroisopropanol at 25°C and at a concentration of 0.10 g/dL. Gel permeation chromatography analysis showed a weight average molecular weight of approximately 60,000 Daltons. Nuclear magnetic resonance analysis confirmed that the resin contained 64 mole percent polymerized L(-)-lactide and 36 mole percent polymerized *epsilon*-caprolactone, with a residual monomer content of about 1.6 percent. The glass transition temperature, Tg, of the dried resin was -17°C, the melting point was 160°C, and the heat of fusion, $\Delta H_m$, was 26 J/g as determined by Differential Scanning Calorimetry using the first heat scan and a heating rate of 10°C/min. Wide Angle X-ray Diffraction (WAXD) analysis revealed that the dried resin contains 34 percent of crystallinity.

## EXAMPLE 2A

### Synthesis of Segmented Block Copolymer Poly(L(-)-lactide-co-*epsilon*-caprolactone) at 72/28 by Mole [Initial Feed Charge of 75/25 Lac/Cap]

Using a conventional 10-gallon stainless steel oil-jacketed reactor equipped with agitation, 5,221 grams of *epsilon*-caprolactone and 5,394 grams of L(-)-lactide were added along with 13.36 grams of diethylene glycol and 9.64 mL of a 0.33M solution of stannous octoate in toluene. After the initial charge, a purging cycle with agitation at a rotational speed of 10 RPM in a downward direction was initiated. The reactor was evacuated to pressures less than 150 mTorr followed by the introduction of nitrogen gas. The cycle was repeated once again to ensure a dry atmosphere. At the end of the final nitrogen purge, the pressure was adjusted to be slightly above one atmosphere. The rotational speed of the agitator was reduced to 7 RPM in a downward direction. The vessel was heated by setting the oil controller at 190°C. When the batch temperature reached 110°C, rotation of the agitator was switched to the upward direction. The reaction continued for 6 hours from the time the oil temperature reached 190°C.

After the completion of the first stage portion of the polymerization, a very small amount of resin was discharged for analysis purposes; selected characterization was performed. The chemical composition of the prepolymer was the same as in Example 1: 45/55 Lac/Cap mole percent with about 2 percent of residual monomer as determined by NMR. The DSC data revealed that the prepolymer was fully amorphous with no crystallinity developed, even after heat treatment. The glass transition temperature was again determined to be -17°C (minus 17°C).

In the second stage, the oil controller set point was raised to 205°C, and 14,384 grams of molten L(-)-lactide monomer was added from a melt tank with an agitator speed of 12.5 RPM in a downward direction for 15 minutes. The agitator speed was then reduced to 7.5 RPM in the downward direction. The oil controller was then decreased to 190°C and the reaction proceeded an additional 3 hours prior to the discharge. At the end of the final reaction period, the agitator speed was reduced to 2 RPM in the downward direction, and the polymer was discharged from the vessel into suitable containers.

The resin was divided into two portions. A minor portion of the divided resin was treated as described in Example 2B. The major portion of the copolymer, 13,930 grams, was subjected to the same grinding, sieving and drying steps described in Example 1 using the following heat/drying treatment: 12 hours at 25°C, 4 hours at 55°C, 4 hours at 75°C, and 12 hours at 110°C, respectively.

The dried resin exhibited an inherent viscosity of 1.52 dL/g, as measured in hexafluoroisopropanol at 25°C and at a concentration of 0.10 g/dL. Gel permeation chromatography analysis showed a weight average molecular weight of approximately 79,000 Daltons. Nuclear magnetic resonance analysis confirmed that the resin contained 72 mole percent polymerized L(-)-lactide and 28 mole percent polymerized *epsilon*-caprolactone with a residual monomer content of about 1.5 percent. The glass transition temperature, $T_g$, of the dried resin was -8°C, the melting point was 169°C, and the heat of fusion, $\Delta H_m$, was 33 J/g as determined by Differential Scanning Calorimetry using the first heat scan procedure and the heating rate of 10°C/min. Wide Angle X-ray Diffraction (WAXD) analysis revealed that the dried resin contained 43 percent of crystallinity.

**EXAMPLE 2B**

**Synthesis of Segmented Block Copolymer Poly(L(-)-lactide-co-*epsilon*-caprolactone) at 74/26 by Mole [Initial Feed Charge of 75/25 Lac/Cap, solid state polymerization final treatment]**

The smaller portion of the discharged resin, 6,900 grams, produced and described in Example 2A above was placed in a nitrogen purged oven and heated for 72 hours at 120°C. This solid state polymerization step was conducted in order to further increase the monomer conversion. After the solid state polymerization treatment, the resin was ground, sieved, and dried using the same procedures described earlier in Examples 1 and 2A.

The dried resin exhibited an inherent viscosity of 1.58 dL/g, as measured in hexafluoroisopropanol at 25°C and at a concentration of 0.10 g/dL. Gel permeation chromatography analysis showed a weight average molecular weight of approximately 83,000 Daltons. Nuclear magnetic resonance analysis confirmed that the resin contained 74 mole percent polymerized L(-)-lactide and 26 mole percent polymerized *epsilon*-caprolactone with a residual monomer content of about 1.0 percent. The glass transition temperature, $T_g$, of the dried resin was -8°C, the melting point was 168°C, and the heat of fusion, $\Delta H_m$, was 39 J/g as determined by Differential Scanning Calorimetry using first heat data and a heating rate of 10°C/min. Wide Angle X-ray Diffraction (WAXD) analysis revealed that the dried resin was 43 percent crystalline.

**EXAMPLE 3A**

**Synthesis of Segmented Block Copolymer Poly(L(-)-lactide-co-*epsilon*-caprolactone) at 74/26 by Mole [Initial Feed Charge of 75/25 Lac/Cap]**

Using a conventional 10-gallon stainless steel oil jacketed reactor equipped with agitation, 5,221 grams of *epsilon*-caprolactone and 2,826 grams of L(-)-lactide were added along with 9.65 grams of diethylene glycol and 9.64 mL of a 0.33M solution of stannous octoate in toluene. The reactor's conditions was identical those in Example 2A, except that the reaction in the first stage lasted for 4 hours from the time the oil temperature reached 190°C.

After the completion of the first polymerization stage, a very small amount of resin was discharged for analysis purposes; selected characterization was performed. The chemical composition of the prepolymer in this case was 30/70 Lac/Cap mole percent with about 3 percent of residual monomer as determined by NMR. The DSC data revealed that the prepolymer was fully amorphous with no crystallinity developed even after heat treatment. The glass transition temperature was found to be lower than that in Examples 1 and 2A, -39°C (minus 39°C), most likely due to the higher *epsilon*-caprolactone content present in the first stage.

In the second stage, the oil controller set point was raised to 205°C, and 16,953 grams of molten L(-)-lactide monomer was added from a melt tank. The oil controller was then decreased to 200°C and the reaction continued an additional 3 hours prior to the discharge.

The major portion of the copolymer, 13,870 grams, was subjected to the same grinding, sieving and drying steps described in Example 1 using the following heat/drying treatment: 12 hours at 25°C, 4 hours at 55°C, 4 hours at 75°C, and 12 hours at 110°C (the same conditions as for Example 2A).

The dried resin exhibited an inherent viscosity of 1.63 dL/g, as measured in hexafluoroisopropanol at 25°C and at a concentration of 0.10 g/dL. Gel permeation chromatography analysis showed a weight average molecular weight of approximately 90,000 Daltons. Nuclear magnetic resonance analysis confirmed that the resin contained 74 mole percent polymerized L(-)-lactide and 26 mole percent polymerized *epsilon*-caprolactone with a residual monomer content of about 1.5 percent. The glass transition temperature, $T_g$, of the dried resin was -34°C, the melting point was 170°C, and the heat of fusion, $\Delta H_m$, was 35 J/g, as determined by Differential Scanning Calorimetry using the first heat data and a heating rate of 10°C/min. Wide Angle X-ray Diffraction (WAXD) analysis revealed that the dried resin was 45 percent crystalline.

## EXAMPLE 3B

### Synthesis of Segmented Block Copolymer Poly(L(-)-lactide-co-*epsilon*-caprolactone) at 76/24 by Mole [Initial Feed Charge of 75/25 Lac/Cap, solid state polymerization final treatment]

The smaller portion of the discharged resin, 8,500 grams, produced and described in Example 3A, was placed in a nitrogen purged oven and heated in a solid state fashion for 72 hours at 120°C. This step was conducted in order to further increase the monomer conversion. After the solid state polymerization treatment, the resin was ground, sieved, and dried using the same procedures described earlier in earlier Examples.

The dried resin exhibited an inherent viscosity of 1.70 dL/g, as measured in hexafluoroisopropanol at 25°C and at a concentration of 0.10 g/dL. Gel permeation chromatography analysis showed a weight average molecular weight of approximately 91,000 Daltons. Nuclear magnetic resonance analysis confirmed that the resin contained 76 mole percent polymerized L(-)-lactide and 24 mole percent polymerized *epsilon*-caprolactone with a residual monomer content of about 1.0 percent. The glass transition temperature, $T_g$, of the dried resin was -34°C, the melting point was 170°C, and the heat of fusion, $\Delta H_m$, was 49 J/g, as determined by Differential Scanning Calorimetry using the first heat data and a heating rate of 10°C/min. Wide Angle X-ray Diffraction (WAXD) analysis revealed that the dried resin was 50 percent crystalline.

## EXAMPLE 4

### Selected Properties of Copolymers of the Present Invention

#### a) Differential Scanning Calorimetry (DSC) and Melt Index (MI) Characteristics

DSC measurements were conducted using a model Q20-3290 calorimeter from TA Instruments (New Castle, DE) equipped with automatic sampler. In individual experiments, the dried, heat treated copolymer resins as described in Examples 1, 2A, 2B, 3A, and 3B were placed into DSC pans, quenched below -60°C, and heated at the constant heating rate of 10°C/min to determine their calorimetric properties (first heat properties); these included the glass transition temperature, $T_g$, the melting point, $T_m$ and the heat of fusion, $\Delta H_m$. From the second heat measurements (resin was melted at 200°C and then quenched below -60°C), values for $T_g$, $T_m$, $T_c$ (crystallization temperature), and $\Delta H_m$ were obtained that are independent from the previous heat treatment history.

Data obtained using calorimetry and melt index measurements are displayed in Table 1.

**Table 1.**

| Melt Index and DSC Results during the First and Second Heat Runs on the Copolymers of the Present Invention | | | | | | | |
|---|---|---|---|---|---|---|---|
| Example | MI (g/10min) | First heat, DSC | | | Second heat, DSC | | |
| | | $T_g$ (°C) | $T_m$ (°C) | $\Delta H_m$ (J/g) | $T_g$(°C) | $T_c$/$T_m$ (°C) | $\Delta H_m$ (J/g) |
| 1 | 0.224 | -17 | 160 | 26 | 12 | 106/160 | 21 |
| 2A | 0.066 | -8 | 169 | 33 | 29 | 125/168 | 25 |
| 2B | 0.052 | -8 | 168 | 39 | 33 | 128/168 | 24 |
| 3A | 0.016 | -34 | 170 | 35 | -32 & 53 | 124/169 | 28 |
| 3B | 0.018 | -34 | 170 | 49 | -35 & 53 | 125/168 | 29 |

The results in Table 1 indicated that the resin of Example 1 exhibited a lower overall crystallinity level (lower $\Delta H_m$ value), and a lower melting point than the rest of the examples. This is most likely due to a higher polymerized *epsilon*-caprolactone content present in this copolymer (36 mole percent) compared to the other resins. As noted before, the resin of Example 1 also has lower weight average molecular weight and IV. With an increase in polymerized lactide level in the structure (Examples 2A-B, 3A-B), the level of crystallinity increases (higher $\Delta H_m$ values), as well as the melting point values. It is very important to note that in all cases only a single $T_g$ is observed after the first heating scans.

The $T_g$ values were all well below room temperature, ranging from minus 8° to minus 34°C; low $T_g$ values may contribute to increased softness of medical devices produced from these materials.

Melt Index (MI) is used as a measure of the melt viscosity of the resins. MI experiments on dried resins of present invention were conducted using an Extrusion Plastometer, Tinius Olsen (Willow Grove, PA, USA) at 175°C with the nominal weight of 2,060 g. The die used in the MI measurements had a capillary with a diameter of approximately 0.023 inches and a length 0.315 inches. The MI data (second column in Table 1) indicate the lowest melt viscosity for Example 1, and the highest for Examples 3A and 3B, which is in agreement with the molecular weight and IV data mentioned earlier.

In order to gain preliminary information on potential fiber characteristics, the copolymers of the present invention were extruded through the Melt Index apparatus (at 215°C), unoriented fiber parts collected, and then subjected to manual heat or cold drawing process until the fibers were fully stretched. Pieces of drawn fibers were examined for handling purposes only. It was found that fibers from all resins from the present invention (Examples 1 to 3B) showed good pliability and softness suitable for making monofilaments.

**b) Isothermal Crystallization Kinetics by DSC**

Crystallization characteristics were assessed. Isothermal crystallization kinetics of the polymers of the present invention were conducted using the Differential Scanning Calorimetry techniques. The dried, heat-treated copolymer resins, as described in Examples 1, 2A, 2B, 3A, and 3B were placed into a DSC pan and completely melted at 200°C for 2 minutes to remove any nucleation sites present in the sample. Subsequently, tested materials were rapidly cooled/quenched (rate -65°C/min) to the desired crystallization temperatures. The isothermal method assumes that no crystallization occurs before the sample reaches the test temperature; the data obtained supported this assumption. Crystallization behavior of the five samples was characterized over a wide range of temperatures, between 40 and 130°C. Isothermal crystallization kinetics (at constant temperature) were monitored as a change in heat flow as a function of time. The isothermal heat flow curve was integrated to determine the crystallinity parameters. It is worth noting that the isothermal DSC runs were made in randomized order to avoid any bias.

The development of crystallinity with time can be accessed from the degree of crystallization,$\alpha$, which is expressed by the ratio

$$\alpha = \frac{\Delta Ht}{\Delta H\infty} = \frac{\int_0^t \frac{dQ}{dt} dt}{\int_0^\infty \frac{dQ}{dt} dt} \qquad (8)$$

where dQ/dt is the respective heat flow; $dH_t$, the partial area between the DSC curve and the time axis at time t; and $dH_\infty$, the total area under the peak and corresponds to the overall heat of crystallization. The degree of crystallization, $\alpha$, is then the crystalline volume fraction developed at time t.

After performing the integration of the heat flow/time curve, the crystallization half-time, $t_{1/2}$, can be determined. The crystallization half-time is the time needed to reach 50 percent crystallinity of the total amount developed during the isothermal run. In order to express crystallization kinetics, a reciprocal crystallization half-time was presented as a function of crystallization temperature. These data are shown in FIG. 1 for resins of Example 1 and 3A. The resins 2A, 2B, and 3B were also examined; both 2A and 2B samples show very similar trend as Example 1. The resins 3A, 3B behaved nearly identically to each other. Several important points can be drawn from the data in FIG. 1. Firstly, all examined resins showed a fast crystallization rate over a wide range of temperatures, especially when compared to random copolymers of the same composition. Fastest kinetics for the examined resins were observed at approximately 95°C.

Interestingly, the plot of Example 1 showed an unusual, second maximum at lower crystallization temperature (around 65°C); the resins of Examples 2A and 2B displayed a second maximum at the same temperature as well. This information may be very useful, for instance, for optimizing extrusion conditions to increase crystallization efficiency during the drawing process. On the other hand, the samples 3A and 3B did not exhibit this lower temperature maximum; here, only a regular bell-shaped curve was observed with the crystallization rates similar to those of Example 1. The lack of low temperature maximum in Figure 1 for 3A and 3B resins may possibly be due to higher second heat $T_g$ values for these copolymers as previously reported in Table 1.

**c) Hydrolysis Profile Data - Comparison with poly(*p*-dioxanone)**

We have assessed the absorbability of the resins of the subject invention by an *in vitro* method. The method was found suitable for estimating synthetic absorbable polyester *in vivo* degradation time. Essentially, the article to be tested is subjected to hydrolysis at a given test temperature and a constant pH. Using pH-stat technology, a solution of week base is added to the test article in an aqueous environment and the amount of base added as a function of time is recorded. *In vivo* absorption time is compared to the generated *in vitro* data, initially with model compounds and a number of commercially available absorbable products to establish a correlation curve.

In vitro absorption time was measured by an automated titration unit (718 Stat Titrino, Brinkmann, Westbury, NY, USA) at 70°C, under constant pH (7.3) in 70 mL of deionized (DI) water using 0.05N NaOH as a base. The weight of materials was about 100 mg. All of the polymer samples were in granular form with 6 pieces chosen for each resin having similar shape and size.

Hydrolysis data indicated that all examined materials hydrolyzed under the test conditions with the rate of disappearance of the copolymers of the present invention being slower than the control sample, poly(*p*-dioxanone) homopolymer. Hydrolysis results are presented in Table 2 in a form of hydrolysis half-time. Hydrolysis half-time is defined as time needed to hydrolyze half of the ester groups originally present. Shorter times suggest faster hydrolysis and vice versa.

**Table 2.**

| Hydrolysis Profile Data of Poly(*p*-dioxanone), PDS Dried Resin and the Final, Heat Treated Copolymers of the Present Invention | | | | | |
|---|---|---|---|---|---|
| Example | Composition (mole %) | Resin shape & size (# of pieces and total weight) | Cryst. % by WAXD | Did Hydrolysis Occur? | Hydrolysis half-time, $t_{1/2}$ (hours) |
| 1 | 64/36 Lac/Cap | Granular, 6 pieces, 96 mg | 34 | Yes | 300 |
| 2A | 72/28 Lac/Cap | Granular, 6 pieces, 96 mg | 43 | Yes | 240 |
| 3A | 74/26 Lac/Cap | Granular, 6 pieces, 97 mg | 45 | Yes | 260 |
| PDS | 100% PDO | Granular, 6 pieces, 97 mg | 55 | Yes | 100 |

It is evident from Table 2 that the inventive copolymers of Examples (1, 2A, and 3A) all exhibited a slower hydrolysis rate than the poly(*p*-dioxanone) homopolymer control, despite the fact that they exhibited lower levels of crystallinity.

## EXAMPLE 6

**Determination of the Average Chain Sequence Length (ACSL) of the Segmented Poly(L(-)-lactide-co-*epsilon*-caprolactone) Segmented Block Copolymers**

The copolymers described in the Examples 1, 2A, 2B, 3A and 3B were subjected to $^{13}C$ NMR analysis (UNITYplus, Varian 400 MHz NMR system) to experimentally determine an average chain sequence length, ACSL for caproyl and lactidyl blocks ($ACSL_{Cap}$ and $ACSL_{LL}$, respectively). The peak assignments and method analysis used were based on the work reported earlier on a similar class of copolymers (Z. Wei et al. / Polymer 50 (2009) 1423-1429). Listed in Table 3 are the final compositions (polymerized lactide /*epsilon*-caprolactone mole ratio), the $ACSL_{LL}$ and $ACSL_{Cap}$ values, the random factors for polymerized lactide and *epsilon*-caprolactone, $RF_{LL}$ and $RF_{cap}$, respectively for the final copolymers of Examples 1, 2A, 2B, 3A and 3B as well as some comparative prior art copolymers. Comparative Copolymer X is a melt prepared random copolymer reported by Wei et al. in 2009 (Z. Wei et al. / Polymer 50 (2009) 1423-1429); Comparative Copolymer Y is a solution prepared random copolymer reported by Vanhoorne, et al. in 1992 (Vanhoorne, et al. / Macromolecules 25 (1992) 37-44; and Comparative Copolymer Z is a melt prepared block copolymer reported by Baimark, et al. in 2005 (Journal of Materials Science: Materials In Medicine 16 (2005) 699-707).

**Table 3**

| $^{13}C$ NMR Data on the Polymers of the Present Invention | | | | | |
|---|---|---|---|---|---|
| Example | Final Composition Lac/ε-Cap (mole %) | $ACSL_{LL}$ | $ACSL_{Cap}$ | $RF_{LL}$ | $RF_{Cap}$ |
| 1 (34% cryst.) | 64/36 | 6.7 | 3.3 | 2.41 | 2.11 |

(continued)

| $^{13}C$ NMR Data on the Polymers of the Present Invention | | | | | | |
|---|---|---|---|---|---|---|
| Example | Final Composition Lac/ε-Cap (mole %) | $ACSL_{LL}$ | $ACSL_{Cap}$ | $RF_{LL}$ | $RF_{Cap}$ |
| 2A (43% cryst.) | 72/28 | 7.3 | 2.3 | 2.04 | 1.66 |
| 2B (43% cryst.) | 74/26 | 7.4 | 2.2 | 1.92 | 1.63 |
| 3A (45% cryst.) | 74/26 | 11.1 | 3.1 | 2.89 | 2.29 |
| 3B (50% cryst.) | 76/24 | 10.8 | 3.3 | 2.59 | 2.51 |
| Comparative Copolymer X (random) | 64/36 | 4.6 | 2.4 | 1.66 | 1.54 |
| Comparative Copolymer Y (random) | 70/30 | 5.1 | 2.2 | 1.53 | 1.54 |
| Comparative Copolymer Z (block) | 79/21 | 8.2 | 2.3 | 1.72 | 1.82 |

Data in Table 3 indicate that for inventive Examples 1, 2A, 2B, 3A, and 3B, the average chain sequence lengths, ACSL, for caproyl and lactidyl blocks ($ACSL_{Cap}$ and $ACSL_{LL}$, respectively), are relatively long vs. the comparative polymers of similar compositions. Shown in FIG. 2 are the relative proportions, on a mole basis, of a variety of 3-member, 4-member, and 5-member sequence combinations; specifically, CCC, LLCC, CCLL, LLCLL, LLLLC, CLLC, CLLLL, and LLLLL. A particularly important sequence combination is the 5-member LLLLL, as it reflects the relative amount of crystallizable lactide in the copolymer, resulting in increased crystallizability and consequently dimensional stability of articles formed therefrom.

The randomness factors for the lactidyl blocks ($RF_{LL}$) of the inventive Examples, as shown in Table 3, are particularly large values. Having high randomness factor parameters indicates much higher blockiness of the lactide sequences in the inventive samples than the comparative examples. A consequence of possessing a high level of blockiness in the copolymers of the current invention is enhanced crystallization rates and ultimate crystallinity levels will be enhanced, leading to better fiber properties.

The novel bioabsorbable copolymers of the present invention can be melt extruded using conventional means into monofilament fibers suitable for medical applications; these applications include the manufacture of monofilament surgical sutures and surgical meshes. The novel bioabsorbable copolymers of the present invention can be melt extruded using conventional means into multifilament fibers suitable for medical applications; these applications include the manufacture of braided surgical sutures and surgical meshes.

The novel bioabsorbable copolymers of the present invention and novel medical devices made from such copolymers are believed and expected to have numerous advantages. The advantages include, but are not limited to, the following: pliability of resulting fibers; extended breaking strength retention profile; the ability to be made into soft monofilaments with low tissue reaction, low tissue pull-through values, and low tissue drag), good moldability and dimensional stability of molded devices. The copolymers can be readily made into long-term absorbable sutures having superior properties, both monofilament and braided constructions.

Although this invention has been shown and described with respect to detailed embodiments thereof, it will be understood by those skilled in the art that various changes in form and detail thereof may be made without departing from the spirit and scope of the claimed invention.

**Further embodiments of the invention**

Embodiment 1. A semicrystalline bioabsorbable segmented copolymer, comprising repeating units of polymerized lactide and polymerized *epsilon*-caprolactone, wherein the mole ratio of polymerized lactide to polymerized *epsilon*-caprolactone is between about 60:40 to 75:25, said copolymer having a first heat $T_g$ as determined by differential scanning calorimetry at a scan rate of 10°C per minute, equal to or less than 0°C, and a crystallinity level of about 25 to about 50 percent, as measured by wide angle X-ray diffraction.

Embodiment 2. The bioabsorbable segmented copolymer of embodiment 1 having an inherent viscosity at least about 0.5 dL/g, as measured in a 0.1 g/dl solution of HFIP at 25°C.

Embodiment 3. The semi-crystalline segmented copolymer of embodiment 1, wherein the copolymer first heat Tg, is below about -5°C.

Embodiment 4. The semi-crystalline segmented copolymer of embodiment 1, having a randomness factor of polymerized lactide in the copolymer of about 1.8 or greater.

Embodiment 5. The semi-crystalline segmented copolymer of embodiment 4, having a randomness factor of polymerized *epsilon*-caprolactone in the copolymer of 1.6 or greater.

Embodiment 6. A suture comprising the copolymer of embodiment 1.

Embodiment 7. The suture of embodiment 6 comprising a monofilament fiber.

Embodiment 8. The monofilament fiber of embodiment 7 having a Young's modulus of less than about 350,000 psi.

Embodiment 9. The suture of embodiment 6 retaining mechanical strength to about 3 months post-implantation.

Embodiment 10. The suture of embodiment 7 retaining mechanical strength to about 3 months post-implantation.

Embodiment 11. The suture of embodiment 6 retaining mechanical strength to about 6 months post-implantation.

Embodiment 12. The suture of embodiment 7 retaining mechanical strength to about 6 months post-implantation.

Embodiment 13. The suture of embodiment 6 retaining mechanical strength to about 9 months post-implantation.

Embodiment 14. The suture of embodiment 7 retaining mechanical strength to about 9 months post-implantation.

Embodiment 15. A bioresorbable copolymer of the structure A-B-A, wherein end-segments A comprise polymerized lactide blocks and the middle segment B comprises a polymerized lactide-co-*epsilon*-caprolactone block, and wherein said middle segment B represents about 25 weight percent to about 60 weight percent of the copolymer.

Embodiment 16. The bioresorbable copolymer of embodiment 15, wherein the mole ratio of the polymerized lactide to polymerized *epsilon*-caprolactone in said copolymer is between about 60:40 to about 75:25.

Embodiment 17. The bioresorbable copolymer of embodiment 15 wherein said A blocks are substantially semi-crystalline, and said B block is substantially amorphous.

Embodiment 18. The bioresorbable copolymer of embodiment 15 wherein said A blocks contain up to about 10 mole percent *epsilon*-caprolactone.

Embodiment 19. The bioresorbable copolymer of embodiment 15 wherein the A blocks contain up to about 10 mole percent glycolide.

Embodiment 20. The bioresorbable copolymer of embodiment 15 wherein the said A blocks contain up to about 10 mole percent glycolide and up to about 10 mole percent *epsilon*-caprolactone.

Embodiment 21. The bioresorbable copolymer of embodiment 15 wherein the B block contains up to about 10 mole percent glycolide.

Embodiment 22. The bioresorbable copolymer of embodiment 15 wherein said A blocks are polymerized lactide representing about 40 weight percent to about 75 weight percent of the copolymer.

Embodiment 23. A bioabsorbable semi-crystalline segmented copolymer comprising the reaction product of:

(a) a pre-polymer formed from polymerizing lactide monomer, and *epsilon*-caprolactone monomer in the presence of an initiator, wherein the mole ratio of lactide to *epsilon*-caprolactone in the prepolymer is between about 45:55 to about 30:70; and

(b) lactide monomer.

Embodiment 24. The copolymer of embodiment 23, wherein the prepolymer is formed in the presence of a suitable amount of a catalyst in addition to the initiator.

Embodiment 25. The bioabsorbable semi-crystalline segmented copolymer of embodiment 23 wherein the prepolymer is substantially amorphous.

Embodiment 26. The bioabsorbable semi-crystalline segmented copolymer of embodiment 23, wherein the inherent viscosity of the prepolymer is between about 0.5 to about 1.5 dl/g as measured in a 0.1 g/dl solution of HFIP at 25°C.

Embodiment 27. The bioabsorbable semi-crystalline segmented copolymer of embodiment 23 wherein the prepolymer is substantially amorphous and the inherent viscosity of the prepolymer is between about 0.5 to about 1.5 dl/g as measured in a 0.1 g/dl solution of HFIP at 25°C.

Embodiment 28. The bioabsorbable semi-crystalline segmented copolymer of embodiment 23 wherein the inherent viscosity of the polymer is between about 0.6 to about 2.5 dl/g as measured in a 0.1 g/dl solution of HFIP at 25°C.

Embodiment 29. The bioabsorbable semi-crystalline segmented copolymer of embodiment 23 wherein the initiator is difunctional.

Embodiment 30. The bioabsorbable semi-crystalline segmented copolymer of embodiment 23 wherein the difunctional initiator is a diol.

Embodiment 31. The copolymer of embodiment 23, wherein the prepolymer is present in the amount of about 25 wt.% to about 60 wt.% of the final copolymer.

Embodiment 32. A bioabsorbable fiber, comprising:

a semicrystalline absorbable segmented copolymer, comprising repeating units of polymerized lactide and polymerized *epsilon*-caprolactone, wherein the mole ratio of polymerized lactide to polymerized *epsilon*-caprolactone is between about 60:40 to 75:25, said copolymer having a first heat $T_g$ as determined by differential scanning calorimetry at a scan rate of 10°C per minute, equal to or less than 0°C, and a crystallinity level of about 25 to about 50 percent, as measured by wide angle X-ray diffraction.

Embodiment 33. A bioabsorbable surgical suture, comprising the fiber of embodiment 32.
Embodiment 34. The suture of embodiment 33, wherein the suture is a monofiliament suture.
Embodiment 35. The suture of embodiment 33, wherein the suture is a multifilament suture comprising at least two fibers.
Embodiment 36. The suture of embodiment 33, additionally comprising an antimicrobial agent.
Embodiment 37. The suture of embodiment 36, wherein the antimicrobial agent comprises triclosan.
Embodiment 38. A surgical mesh, comprising the fiber of embodiment 32.
Embodiment 39. The surgical suture of embodiment 33, additionally comprising at least one barb.
Embodiment 40. A bioabsorbable medical device, said medical device comprising the copolymer of embodiment 1.
Embodiment 41. The medical device of embodiment 40, wherein the device is selected from the group consisting of tissue repair fabrics, suture anchors, stents, orthopedic implants, meshes, staples, tacks, fasteners, and suture clips.
Embodiment 42. The medical device of embodiment 41 wherein the tissue repair fabric is a melt blown nonwoven fabric.
Embodiment 43. The medical device of embodiment 41 wherein the tissue repair fabric is an electrostatically spun fabric.
Embodiment 44. The medical device of embodiment 40, additionally comprising an antimicrobial agent.
Embodiment 45. The medical device of embodiment 40, wherein the antimicrobial agent comprises triclosan.
Embodiment 46. A medical device comprising the polymer of embodiment 1, wherein the medical device is injection molded and deformable.
Embodiment 47. The medical device of embodiment 46 further comprising a therapeutic agent.
Embodiment 48. A medical device comprising the polymer of embodiment 1, wherein the medical device is a microcapsule or a microsphere.
Embodiment 49. The medical device of embodiment 48 further comprising a therapeutic agent.

## Claims

**1.** A bioresorbable copolymer of the structure A-B-A, wherein end-segments A comprise polymerized lactide blocks and the middle segment B comprises a polymerized lactide-co-*epsilon*-caprolactone block, and wherein said middle segment B represents about 25 weight percent to about 60 weight percent of the copolymer.

**2.** The bioresorbable copolymer of claim 1, wherein the mole ratio of the polymerized lactide to polymerized *epsilon*-caprolactone in said copolymer is between about 60:40 to about 75:25.

**3.** The bioresorbable copolymer of claim 1 wherein said A blocks are substantially semi-crystalline, and said B block is substantially amorphous.

**4.** The bioresorbable copolymer of claim 1 wherein said A blocks

(a) contain up to about 10 mole percent *epsilon*-caprolactone; or
(b) contain up to about 10 mole percent glycolide; or
(c) contain up to about 10 mole percent glycolide and up to about 10 mole percent *epsilon*-caprolactone; or
(d) are polymerized lactide representing about 40 weight percent to about 75 weight percent of the copolymer.

**5.** The bioresorbable copolymer of claim 1 wherein the B block contains up to about 10 mole percent glycolide.

**6.** A bioabsorbable medical device, said medical device comprising the bioresorbable copolymer of claim 1.

**7.** The medical device of claim 6, wherein the device is selected from the group consisting of tissue repair fabrics, suture anchors, stents, orthopedic implants, meshes, staples, tacks, fasteners, and suture clips; optionally wherein the tissue repair fabric is a melt blown nonwoven fabric or an electrostatically spun fabric.

**8.** The medical device of claim 6, wherein the medical device is injection molded and deformable.

**9.** The medical device of claim 6, wherein the medical device is a microcapsule or a microsphere.

**10.** A suture comprising the bioresorbable copolymer of claim 1.

**11.** The suture of claim 10 comprising a monofilament fiber, optionally wherein the monofilament fiber has a Young's modulus of less than about 350,000 psi.

**12.** The suture of claim 10 or 11 retaining mechanical strength to either about 3 months post-implantation, about 6 months post-implantation, or about 9 months post-implantation.

**13.** The medical device or suture of any of claims 6 to 12, further comprising an active ingredient or comprising a coating containing an active ingredient, such as antimicrobials, antibiotics, therapeutic agents, hemostatic agents, radio-opaque materials, tissue growth factors and combinations thereof; optionally wherein the active ingredient comprises Triclosan, PHMB, silver, silver derivatives or combinations thereof.

**14.** Triclosan, PHMB, silver, silver derivatives or combinations thereof for use as an antimicrobial administered with the medical device or suture of any of claims 6 to 13.

**15.** A semicrystalline bioabsorbable segmented copolymer, comprising repeating units of polymerized lactide and polymerized *epsilon*-caprolactone, wherein the mole ratio of polymerized lactide to polymerized *epsilon*-caprolactone is between about 60:40 to 75:25, said copolymer having a first heat $T_g$ as determined by differential scanning calorimetry at a scan rate of 10°C per minute, equal to or less than 0°C, and a crystallinity level of about 25 to about 50 percent, as measured by wide angle X-ray diffraction.

**FIG. 1.**

**Isothermal Crystallization Kinetics, as measured by Differential Scanning Calorimetry, of the Final Copolymers of Examples 1 and 3A.**

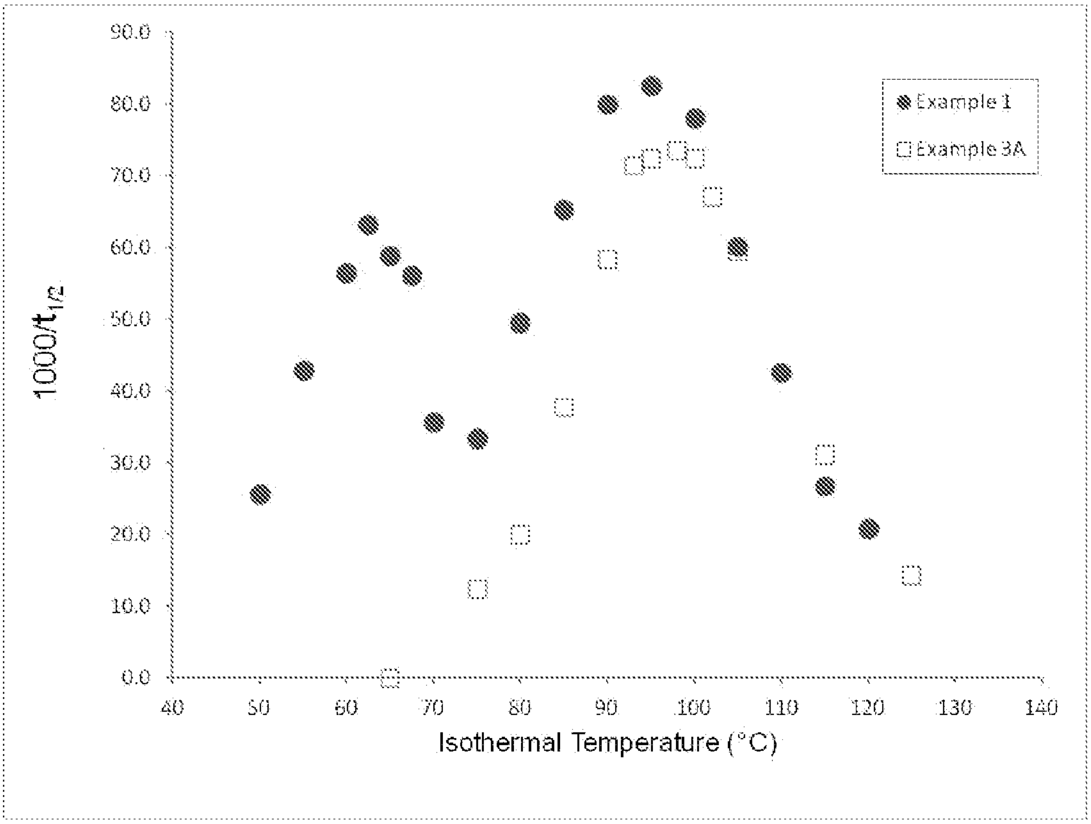

FIG. 2.

**A Histogram of Sequence Distribution Results for the final Inventive Copolymers of Examples 1, 2A, 2B, 3A and 3B as measured by $^{13}C$ NMR.**

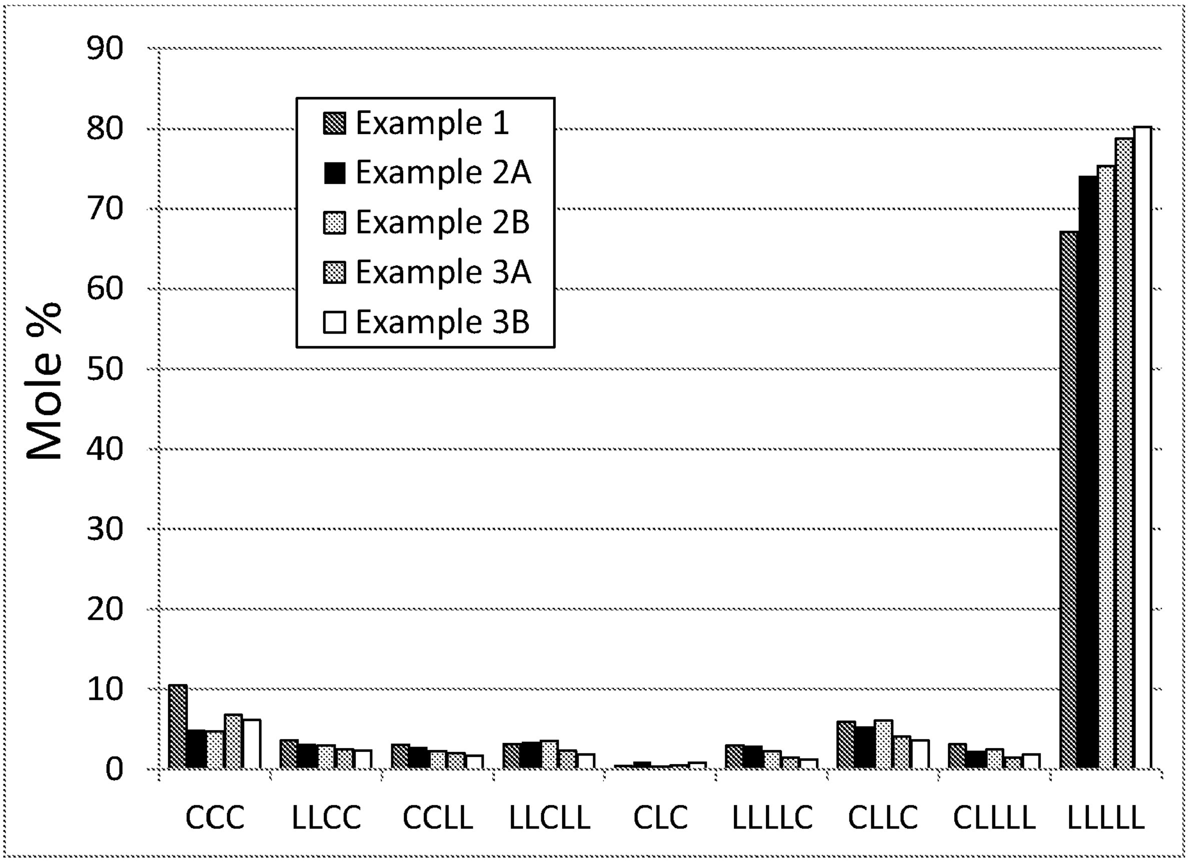

Europäisches Patentamt
European Patent Office
Office européen des brevets

# EUROPEAN SEARCH REPORT

Application Number
EP 17 16 9121

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | DE 43 00 420 A1 (BASF AG [DE]) 14 July 1994 (1994-07-14) * claims 1-6; example 2 * * page 2, line 64 - page 3, line 5 * * page 4, paragraphs 2,4 * | 1-15 | INV. C08G63/00 A61L31/10 |
| X | BAIMARK Y.: POLYMERS FOR ADVANCED TECHNOLOGIES, vol. 16, 28 February 2005 (2005-02-28), pages 332-337, XP002711217, DOI: 101002/pat588 * the whole document * | 1-15 | |
| X | BAIMARK Y.: JOURNAL OF APPLIED POLYMER SCIENCE, vol. 106, 17 August 2007 (2007-08-17), pages 3315-3320, XP002711218, DOI: 10.1002/app.26993 * the whole document * | 1-15 | |
| A | "ISO 11357-2- Plastics. Differential scanning calorimetry (DSC). Part 2: Determination of glass transition temperature", INTERNATIONAL STANDARD ISO/IEC, XX, XX, vol. 11357, 15 March 1999 (1999-03-15), pages 1-5, XP009129728, * pages 2,3 * | 1-15 | TECHNICAL FIELDS SEARCHED (IPC) C08G A61L |
| X | WO 2011/059408 A1 (UNIV NANYANG TECH [SG]; VENKATRAMAN SUBRAMANIAN [SG]; ABADIE MARC [SG]) 19 May 2011 (2011-05-19) * claims 1,5,6,22,25,26,34 * * paragraphs [0023], [0024] - [0028], [0030], [0044], [0054] * * examples 1,2; tables 1-6 * | 1,15 | |

1

The present search report has been drawn up for all claims

| Place of search | Date of completion of the search | Examiner |
|---|---|---|
| Munich | 29 May 2017 | Scheid, Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 16 9121

This annex lists the patent family members relating to the patent documents cited in the above-mentioned European search report. The members are as contained in the European Patent Office EDP file on
The European Patent Office is in no way liable for these particulars which are merely given for the purpose of information.

29-05-2017

| Patent document cited in search report | Publication date | Patent family member(s) | Publication date |
|---|---|---|---|
| DE 4300420 A1 | 14-07-1994 | NONE | |
| WO 2011059408 A1 | 19-05-2011 | EP 2498835 A1 | 19-09-2012 |
| | | US 2012283391 A1 | 08-11-2012 |
| | | WO 2011059408 A1 | 19-05-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5047048 A
- US 5314989 A
- US 5522841 A
- US 5705181 A
- US 5133739 A
- US 20090264040 A1
- US 5797962 A
- US 6342065 B
- US 6712838 B


### Non-patent literature cited in the description


- **Y. BAIMARK ; R. MOLLOY ; N. MOLLOY ; J. SIRIPITAYANANON ; W. PUNYODOM ; M. SRIYAI.** *Journal of Materials Science: Materials In Medicine,* 2005, vol. 16, 699-707
- **HARWOOD, H. J. ; RITCHEY, W. M.** *Polymer Lett.,* 1964, vol. 2, 601
- **Z. WEI et al.** *Polymer,* 2009, vol. 50, 1423-1429
- **VANHOORNE et al.** *Macromolecules,* 1992, vol. 25, 37-44
- **BAIMARK et al.** *Journal of Materials Science: Materials In Medicine,* 2005, vol. 16, 699-707